# EUROPÄISCHE PATENTSCHRIFT

(11) **EP 0 916 326 B1**
(45) Veröffentlichungstag und Bekanntmachung des Hinweises auf die Patenterteilung: **16.01.2002**
(21) Anmeldenummer: 98250362.5
(22) Anmeldetag: 15.10.1998
(51) Int. Cl.: A61F 13/15

(54) **Vorrichtung und Verfahren zum Konturschneiden einer Materialbahn**
Method and apparatus for contour cutting of a continuous web
Procédé et appareil pour la découpe de contours d'une bande

(30) Priorität: 18.11.1997 DE 19751041
(43) Veröffentlichungstag der Anmeldung: 19.05.1999
(73) Patentinhaber: SCHOBER GmbH Werkzeug- und Maschinenbau, D-71735 Eberdingen (DE)
(72) Erfinder: Wittmaier, Klaus, 71665 Vaihingen/Enz - Aurich (DE)
(74) Vertreter: Dreiss, Fuhlendorf, Steimle & Becker

(56) Entgegenhaltungen:
- EP-A- 0 396 050
- EP-A- 0 768 073
- DE-A- 3 911 834
- DE-A- 19 615 560
- US-A- 5 034 007
- US-A- 5 695 846

## Beschreibung

Die Erfindung betrifft eine Vorrichtung und ein Verfahren zum Konturschneiden einer Materialbahn und ein mit diesem Verfahren bzw. dieser Vorrichtung hergestelltes Produkt.

Derartige Vorrichtungen bzw. Verfahren sind aus der EP-A-0,396,050, aus der EP-A-0,768,073 und der US-A-5,034,007 bekannt. Auch die US-A-5,695,846 beschreibt ein Verfahren zur Herstellung von Sand-Uhrglas-förmigen Absorptionskörpern.

Bekannte Schneideinrichtungen weisen folgende Nachteile auf:
a) Bei großen Arbeitsbreiten müssen große und schwere Schneidwalzen eingesetzt werden. Diese sind überproportional teuer.
b) Bei Abstumpfung des Schneidwerkzeuges oder Beschädigung eines Schneideteils, muss immer die ganze Schneidwalze ausgetauscht werden.
c) Die bewegten Masssen sind groß und erfordern eine entsprechende Energie beim Beschleunigen und bei Verzögerung.
d) Die Schnittkraft (Anpressdruck) kann nur für das gesamte Schneidwerkzeug eingestellt werden.

Diese Nachteile werden durch die erfindungsgemäße Vorrichtung gelöst.

Nachfolgend wird die Funktion einer ersten Ausführungsform der erfindungsgemäßen Vorrichtung beschrieben.

Eine breite Warenbahn wird der Vorrichtung zugeführt und von Zugwalzen übernommen. Anschließend durch mehrere Längsschneidemesser in einzelne, schmale Bahnen getrennt und durch weitere Zugwalzen transportiert. Die Bahnen werden getrennt und auf zwei oder mehr verschiedene Ebenen getrennt, dort wieder von Zugwalzen übernommen und mittels einer Schneidgegenwalze und einer nachgelagerten Zugwalze.

Alle Zugwalzen sind schwenkbar. Der Konturschnitt wird von einzelnenen Schneidwalzen, z.B. einem rotativen Schneidwerkzeug gegen die Schneidgegenwalze angebracht. Die Kontur wie auch die Schnitt/Steg-Teilung kann beliebig sein. Das rotative Schneidwerkzeug wie auch die Längsschneidwerkzeuge sind seitlich einstellbar und daher für verschiedene Breiten einsetzbar.

Diese Vorrichtung weist folgende Vorteile auf:
- Breite Warenbahnen können getrennt werden und auf beliebig vielen Ebenen verarbeitet werden
- Die Anzahl der Warenbahnen kann beliebig sein.
- Die rotativen Schneidwerkzeuge sind einfach und gegenüber großen Walzen klein und kostengünstig.
- Die rotativen Schneidwerkzeuge sind seitlich einstellbar.
- Die rotativen Schneidwerkzeuge sind leicht demontierbar und können gegen Werkzeuge mit anderer Kontur ausgetauscht werden.
- Jedes rotative Schneidwerkzeug ist einzeln bezüglich des Schneiddrucks (Kraft) einstellbar.
- Jedes rotative Schneidwerkezug ist einzeln bezüglich des Schneiddrucks (Kraft) einstellbar.
- Die Schneidkontur kann umlaufend, aber auch unterbrochen sein.

Nachfolgend wird die Funktion einer zweiten Ausführungsform der erfindungsgemäßen Vorrichtung beschrieben.

Eine breite Warenbahn wird der Vorrichtung zugeführt und von Zugwalzen übernommen.

Die Warenbahn wird durch mehrere Längsschneidenmesser in einzelne, schmale Bahnen getrennt.

Die Längsschneidefunktion kann aber auch Bestandteil der nachfolgenden Ultra-Schall Schneideeinrichtung sein.

Weiter wird die Materialbahn von einem zugwalzenpaar übernommen und transportiert.

Dann kann die Warenbahn jetzt auf zwei verschiedene Ebenen verteilt werden.

Mit dem rotativen Schneidwerkzeug und der UltraschallEinrichtung können nun beliebige Konturen in die Vliesbahn als Schnitte eingebracht werden und das Material wird anschließend abgeführt.

Diese Vorrichtung weist folgende Vorteile auf:
- Breite Warenbahnen können getrennt werden und auf beliebig vielen Ebenen verarbeitet werden.
- Die Anzahl der Warenbahnen kann beliebig sein.
- Mit den Ultra-Schall-Einrichtungen kann durch Veränderung der Amplitude an der Sonotrode der Schneidhub eingestellt werden und so der Verschleiß kompensiert werden.
- Der Abstand zwischen Sonotrode und Werkzeug ist einstellbar und kann nachgeregelt werden, indem die Aufnahme der Ultraschalleinrichtung gegenüber der rotativen Schneidwalze verändert wird.

Mit dieser Vorrichtung werden folgende Nachteile von bekannten Schneideinrichtungen für breite Warenbahnen vermieden:
a) Bei Abstumpfung der Schneidwerkzeuge können diese nur bedingt zum Gegenwerkzeug verstellt werden und verlieren so ihre Schneidfähigkeit.
b) Bei Schneideinrichtungen mit Schneidwalze und Gegenschneidwalze sind die bewegten Massen groß und erfordern einen entsprechenden Energieaufwand beim Beschleunigen und Verzögern.
c) Die Schnittkraft kann nur bedingt oder gar nicht eingestellt werden.

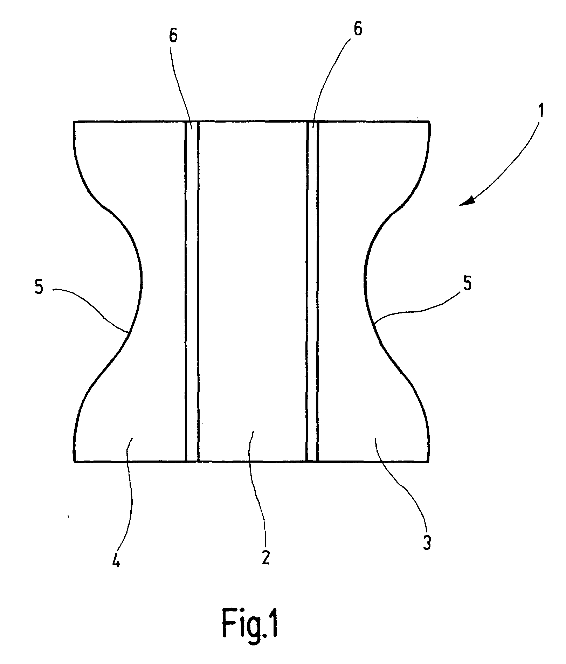

Die Figur 1 zeigt ein mit der erfindungsgemäßen Vorrichtung und/oder dem erfindungsgemäßen Verfahren hergestelltes Produkt, z.B. eine Windel 1. Diese Windel 1 weist einen mittleren Bereich 2 und zwei Seitenteile 3 und 4 auf, in denen die Beinausschnitte 5 vorgesehen sind. Die Seitenteile 3 und 4 sind in einem Bereich 6 mit dem mittleren Bereich 2 verbunden, z.B. verklebt oder verschweißt.

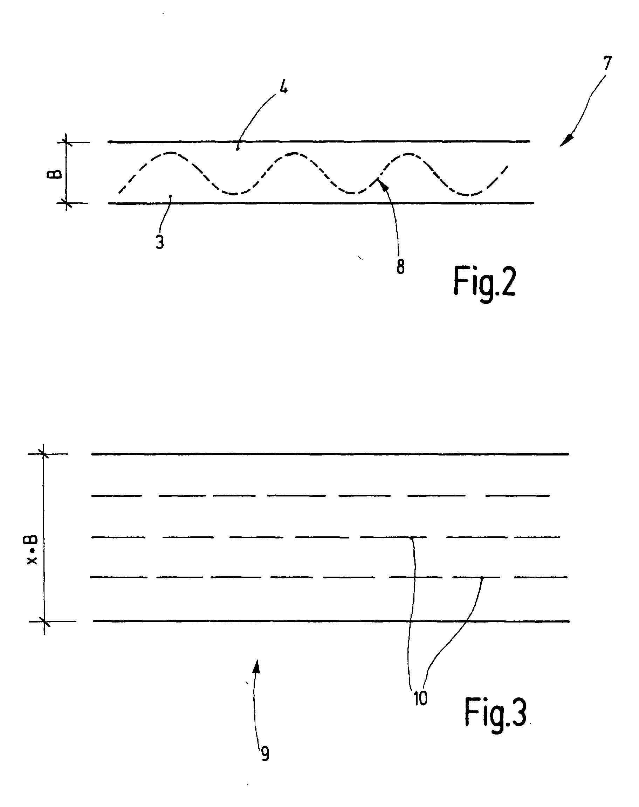

Die Figur 2 zeigt ein Endprodukt 7 mit einer Breite B und beliebiger Materialdicke. Mit 8 ist ein Konturschnitt angedeutet, dessen Verlauf beliebig sein kann und der eine bliebige Anzahl von Haltestegen (nicht näher dargestellt) aufweisen kann. Die Größe der Haltestege ist ebenfalls frei wählbar.

Die Figur 3 zeigt das Ausgangsmaterial 9, aus welchem mehrere Endprodukte herstellbar sind. Das Ausgangsmaterial 9 besitzt eine x-fach Breite und wird über Schnitte 10 in die entsprechende Anzahl Endprodukte aufgeteilt.

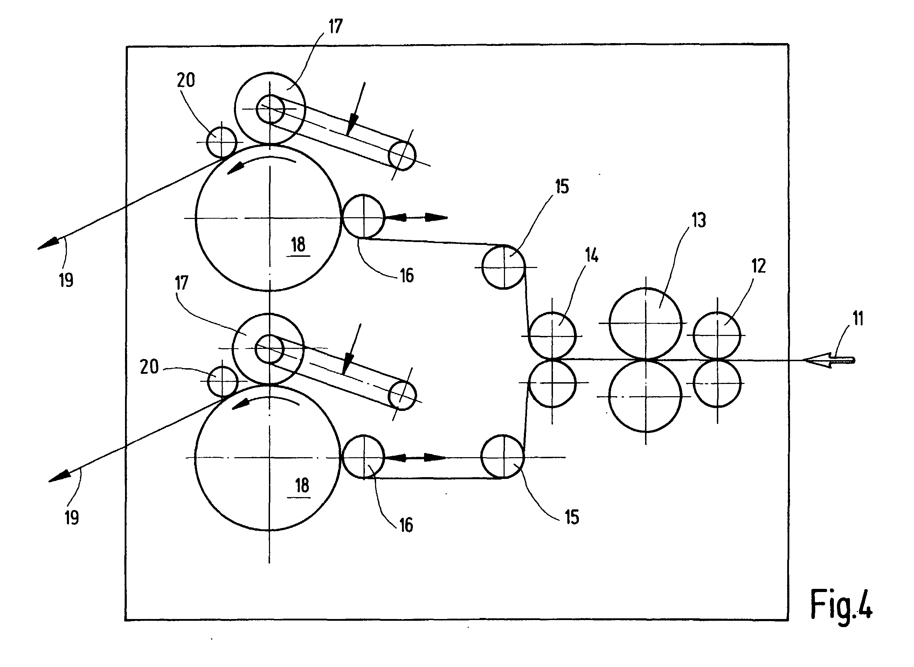

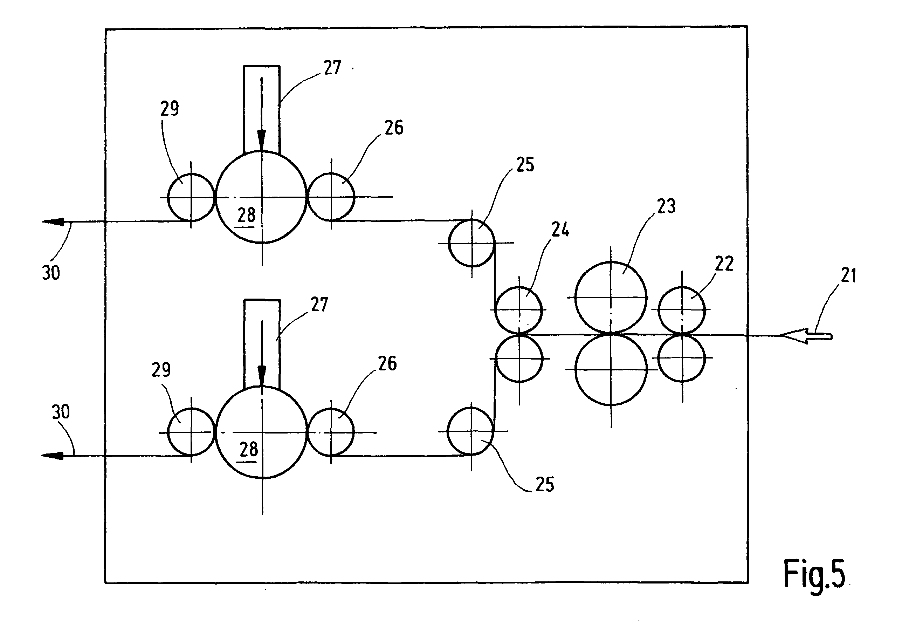

Die Figuren 4 und 5 zeigen zwei Ausführungsbeispiele der erfindungsgemäßen Vorrichtung.

### Bezugszeichenliste

- 1.: Windel
- 2.: mittlerer Bereich
- 3.: Seitenteil
- 4.: Seitenteil
- 5.: Beinausschnitt
- 6.: Befestigungsbereich
- 7.: Streifen, Endprodukt
- 8.: Konturschnitt
- 9.: Ausgangsmaterial, Materialbahn
- 10.: Schnittlinien
- 11.: Vliesbahn (mehrfache Breite von 19.)
- 12.: Einzugswellenpaar
- 13.: Längsschneidewerkzeuge
- 14.: Zugwellenpaar zum Trennen der Materialstreifen
- 15.: Umlenkrollen
- 16.: Zugwellen, beweglich zum Andrücken an 18. und zum Transport der einzelnen Vliesbahnen
- 17.: Rotatives Schneidwerkzeug, anschwenkbar an 18., angetrieben oder freilaufend
- 18.: Angetriebener Gegenschneidzylinder
- 19.: Einzelne Warenbahnen, deren ... Breite 11. entspricht, mit Konturschnitt
- 20.: Auszugsrollen ausgeführt wie 16.
- 21.: Vliesbahn
- 22.: Einzugswellenpaar
- 23.: Längsschneidewerkzeuge
- 24.: Zugwellenpaar, zum Trennen der Materialstreifen
- 25.: Umlenkrollen
- 26.: Zugwellen, beweglich zum Andrücken an 28. und zum Transport der einzelnen Vliesbahnen
- 27.: Ultra-Schallwerkzeug bestehend aus Vorschubeinheit, Konverter, Transformation, Sonotrode, Halter, Sensor und externem Generator und Regeleinheit
- 28.: Rotatives Schneidwerkzeug, mit beliebiger Schneidenkontur, evtl. auch Schnitt/Stegteilung
- 29.: Auszugswelle änlich 26.
- 30.: Einzelne Warenbahnen

## Patentansprüche

1. Vorrichtung zum Konturschneiden einer Materialbahn mit
- einer Einrichtung zum Aufteilen der Materialbahn in Längsstreifen;
- einer Einrichtung zum Einbringen der Längsstreifen in verschiedene Ebenen; und
- einer Einrichtung zum Einbringen eines Konturschnitts in die Längsstreifen in jeder Ebene.

2. Vorrichtung nach Anspruch 1, **dadurch gekennzeichnet, dass** die Einrichtung zum Einbringen des Konturschnittes eine Schneidwalze mit Gegendruckzylinder oder eine Ultraschall-Schneideinrichtung aufweist.

3. Vorrichtung nach Anspruch 1, **dadurch gekennzeichnet, dass** die Einrichtung zum Einbringen des Konturschnittes bezüglich des Schneiddruckes und/oder seitlich bezüglich des Längsstreifens einstellbar ist.

4. Vorrichtung nach Anspruch 1, **dadurch gekennzeichnet, dass** die Einrichtung zum Einbringen des Konturschnittes an die Breite des Längsstreifens angepasst ist.

5. Vorrichtung nach Anspruch 1, **dadurch gekennzeichnet, dass** die Einrichtung zum Einbringen des Konturschnittes schmäler ist, als das Längsschneidwerkzeug.

6. Verfahren zum Konturschneiden einer Materialbahn, bei dem die Materialbahn in Längsstreifen aufgeteilt wird, die Längsstreifen in verschiedene Ebenen verbracht werden und die Längsstreifen in den verschiedenen Ebenen mit einem Konturschnitt versehen werden.

7. Verfahren nach Anspruch 6, **dadurch gekennzeichnet, dass** die Einrichtung zum Einbringen des Konturschnittes bezüglich des Längsstreifens eingestellt wird.

## Claims

1. Device for contour cutting a web of material with
- a device for dividing the web of material into longitudinal strips;
- a device for introducing the longitudinal strips into various planes; and
- a device for introducing a contour cut into the longitudinal strips in each plane.

2. Device according to claim 1, **characterised in that** the device for introducing the contour cut has a cutting roller with counter-pressure cylinder or an ultrasonic cutting device.

3. Device according to claim 1, **characterised in that** the device for introducing the contour cut can be adjusted with respect to the cutting pressure and/or laterally with respect to the longitudinal strip.

4. Device according to claim 1, **characterised in that** the device for introducing the contour cut is adapted to the width of the longitudinal strip.

5. Device according to claim 1, **characterised in that** the device for introducing the contour cut is narrower than the longitudinal cutting tool.

6. Process for contour cutting a web of material in which the web of material is divided into longitudinal strips, the longitudinal strips are moved into various planes and the longitudinal strips in the various planes are provided with a contour cut.

7. Process according to claim 6, **characterised in that** the device for introducing the contour cut is adjusted with respect to the longitudinal strip.

## Revendications

1. Appareil pour réaliser une découpe de contour dans une bande de matière, comportant
- un dispositif pour partager la bande de matière en rubans allongés;
- un dispositif pour disposer les rubans allongés dans différents plans ; et
- un dispositif pour disposer dans chaque plan une découpe de contour effectuée dans les rubans allongés.

2. Appareil selon la revendication 1, **caractérisé en ce que** le dispositif pour disposer la découpe de contour comporte un rouleau de coupe doté d'un cylindre antagoniste, ou bien un dispositif de coupe à ultrasons.

3. Appareil selon la revendication 1, **caractérisé en ce que** le dispositif pour disposer la découpe de contour est réglable suivant la pression de coupe et/ou latéralement par rapport au ruban allongé.

4. Appareil selon la revendication 1, **caractérisé en ce que** le dispositif pour disposer la découpe de profil est adaptée à largeur du ruban allongé.

5. Appareil selon la revendication 1, **caractérisé en ce que** le dispositif pour disposer la découpe de profil est plus petit que l'outil de coupe longitudinal.

6. Procédé pour effectuer une découpe de profil dans une bande de matière, dans lequel la bande de matière est partagée en rubans allongés, les rubans allongés sont disposés dans différents plans, et les rubans allongés dans les différents plans sont pourvus d'une découpe de profil.

7. Procédé selon la revendication 6, **caractérisé en ce que** le dispositif pour disposer la découpe de profil est réglé par rapport au ruban allongé.
